# EUROPÄISCHE PATENTSCHRIFT

(11) **EP 0 432 566 B1**
(45) Veröffentlichungstag und Bekanntmachung des Hinweises auf die Patenterteilung: **29.06.1994**
(21) Anmeldenummer: 90122609.2
(22) Anmeldetag: 27.11.1990
(51) Int. Cl.: A61F 13/04

(54) **Gehgipssohle**
Sole for walking cast
Semelle pour plâtre de marche

(30) Priorität: 15.12.1989 DE 3941416
(43) Veröffentlichungstag der Anmeldung: 19.06.1991
(73) Patentinhaber: Beiersdorf Aktiengesellschaft, 20245 Hamburg (DE)
(72) Erfinder: Lenzen, Franz Richard, W-4150 Krefeld (DE)

(56) Entgegenhaltungen:
- DE-C- 425 470
- DE-C- 593 479
- DE-C- 2 740 400
- DE-U- 7 412 228
- FR-A- 1 125 054
- US-A- 2 759 275
- US-A- 3 613 674

## Beschreibung

Die Erfindung betrifft eine Gehgipssohle mit einem Sohlenkörper, in dem mehrere Durchtritte zur Querführung eines darin eingezogenen Gurtbandsystems zwischen den Seitenrändern des Sohlenkörpers vorgesehen sind, wobei die Seitenränder vorragend ausgebildet sind und wobei am hintenseitigen Ende eine nach oben vorstehende Fersenstütze mit vorragenden Seitenrändern angeformt ist. Solche Gehgipssohlen werden unter einem Gehgips - oder auch unter einem Liegegips - angeschnallt und haben den Vorteil, daß der Gehgips selbst nicht verschmutzt und daß ein besseres Abrollen erreicht wird.

Eine derartige Gehgipssohle ist beispielsweise aus der DE-C-2740̸40̸0̸ bekannt.

Es sind verschiedene Ausführungsformen solcher Gehgipssohlen bekannt. So ist beispielsweise in der DE-PS 922 0̸10̸ eine Gehgipssohle offenbart, die allein aus einem Gummisohlenkörper besteht. Im vorderen Bereich ist ein Gurtbandsystem zum Anschnallen des Gummisohlenkörpers vorgesehen. Im mittleren und hinteren Bereich des Sohlenkörpers sind untenseitig Nuten eingeformt, die sich teilweise kreuzen. In diese Nuten können Mullbinden oder dergleichen eingelegt werden, die bis zu dem Gipsverband heraufgeführt und in diesen eingegipst werden. Nachteilig ist dabei, daß diese Gehgipssohle nicht abgenommen werden kann, es sei denn, man schneidet die Mullbinden durch und befestigt die Gehgipssohle anschließend mit neuen Mullbinden, die dann wieder eingegipst werden müssen.

In der CH-PS 293 292 ist eine Gehgipssohle beschrieben, die einen nach unten gewölbten Sohlenkörper aufweist, der zwei hintereinanderliegende Durchtritte hat. Durch diese Durchtritte ist jeweils ein Gurtband zur Befestigung der Gehgipssohle am Gehgips eingezogen. Damit die Gehgipssohle nicht vom Gehgips abrutscht, ist zusätzlich in den Gehgips ein Bügelträger eingegipst, der nach unten vorsteht und Aufnahme in eine daran angepaßte Nut im Sohlenkörper findet. Nur mit diesem Bügelträger liegt der Fuß des Patienten auf der Gehgipssohle auf. Nachteilig ist, daß der Bügelträger sehr genau eingegipst werden muß, damit die Gehgipssohle anschließend die richtige Stellung zum Fuß hat. Außerdem ist der Sohlenkörper sehr voluminös, was zu einer starken Verlängerung des geschienten Beins und damit zu einer starken Gehbehinderung führt.

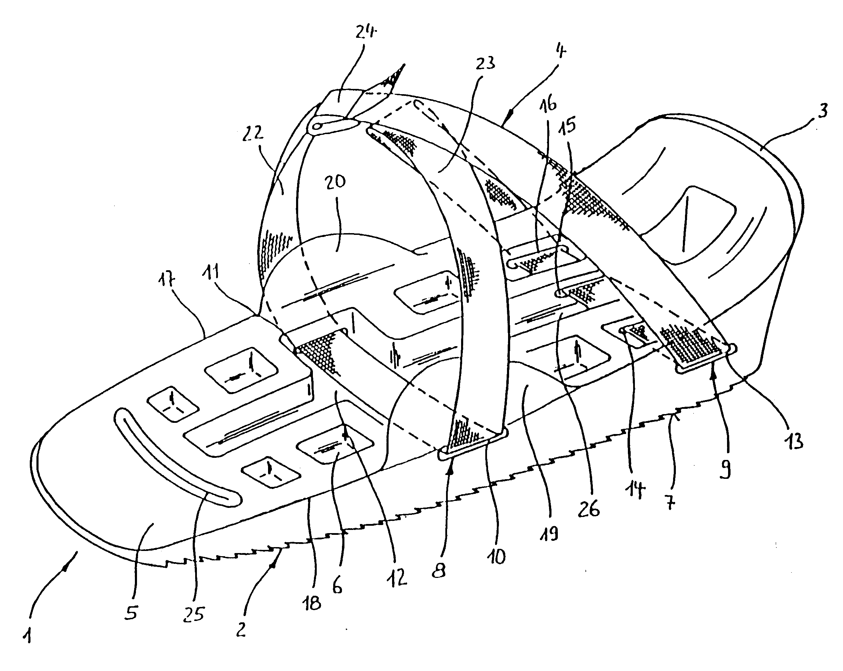

In "Zeitschrift für Orthopädie und ihre Grenzgebiete", 1968, Band 10̸4, Seiten 10̸9 bis 116 sind verschiende Ausführungen von Gehgipssohlen dargestellt. In Abbildung 7c ist eine Gehgipssohle offenbart, die am hinteren Ende eine Fersenstütze aufweist. Sowohl an der Fersenstütze als auch im vorderen Bereich des Sohlenkörpers sind Gurtbänder angeordnet, mit denen die Gehgipssohle an einen Gehgips angeschnallt werden kann. Da die Auflagefläche des Sohlenkörpers nahezu eben ist, hat man trotz des vorderen Gurtbandes einen schlechten Halt in diesem Bereich. Auch das hintere Gurtband verläuft nicht optimal, da es den Gehgips im Fersenbereich wegen seines im wesentlichen horizontalen Verlaufes nicht am Abheben vom Sohlenkörper hindert.

Eine Weiterentwicklung dieser Gehgipssohle ist in der DE-PS 27 40̸ 40̸0̸ offenbart. Sie hat einen Sohlenkörper und eine hinten angeformte Fersenstütze. Im vorderen Bereich und in der Fersenstütze sind Durchtritte vorgesehen, in die jeweils ein Gurtband eingezogen ist. Außerdem sind die Ränder des Sohlenkörpers vorragend ausgeführt. Durch diese Ränder soll erreicht werden, daß der Fuß im Bereich des Sohlenkörpers einwandfrei festgelegt wird. Das an der Fersenstütze in Richtung derselben verlaufende Gurtband führt zu einer starken Erhöhung der Festigkeit der Stütze in diesem Bereich, wodurch Materialeinsparungen möglich sind. Nachteilig ist auch hier die Gurtbandführung. Das hintere Gurtband kann wegen seiner im wesentlichen waagerechten Führung nicht ein Abheben des Fersenbereichs des Gehgipses verhindern. Außerdem muß die seitliche Führung im wesentlichen von der Fersenstütze aufgenommen werden, weshalb deren Ränder vorragend ausgebildet sind. Auch im vorderen Bereich ist die Festlegung des Gehgipses ungenügend.

Damit die Gehgipssohle auch bei Gehgipsen eingesetzt werden kann, die etwas breiter sind als der Abstand der Seitenränder des Sohlenkörpers, sind diese nur geringfügig vorragend ausgebildet. Entsprechend ist die Führung in diesem Bereich unbefriedigend, und zwar sowohl bei passendem Gehgips als auch bei einem Gehgips mit seitlichem Überstand.

Daneben sind Gehhilfen bekannt, die als Gehgipsgaloschen in Form eines Überschuhs ausgebildet sind, die über den Gehgips ziehbar sind (DE-AS 24 43 416). Die Seitenwandungen des Überschuhs sind nach innen geneigt, so daß sie den Gehgips weitgehend umfassen. Solche Gehgipsgaloschen haben sich jedoch nicht durchsetzen können, da der Gehgips keine feste Verbindung mit der Galosche eingeht, der Gehgips also in dieser verrutschen kann.

Die US-A-2 759 275 betrifft einen Überschuh ohne Fersenteil für den vorderen Bereich von Damenschuhen, wobei Führungsstege vorgesehen sind, über die ein Gurtbandsystem verläuft. Die Führungsstege weisen jedoch eine über die Höhe gleichbleibende Flexibilität auf, da ihre Dicke über die Höhe konstant ist. Ein derartiger Überschuh würde sich nicht als Gehsohle unter Gipsverbänden eignen, da sie keine ausreichende Stützung und Führung für den Verband bieten.

Der Erfindung liegt die Aufgabe zugrunde, eine Gehgipssohle der eingangs genannten Art so zu gestalten, daß die Gehgipssohle wesentlich besser am Gehgips festgelegt wird.

Diese Aufgabe wird erfindungsgemäß gelöst durch eine Gehgipssohle gemäß Anspruch 1.

Durch die zusätzlichen, von den Seitenrändern hochstehenden, flexiblen Führungsstege wird die Festlegung des Gehgipses an der Gehgipssohle wesentlich verbessert. Die Führungsstege sorgen im vorderen Bereich für eine Festlegung des Gehgipses, wobei sie hinsichtlich ihrer Flexibilität bzw. Biegsamkeit so ausgebildet sind, daß sie einerseits ein seitliches Herausrutschen des Gehgipses zuverlässig verhindern, also hierfür steif genug sind, andererseits sich jedoch an die außenseitige Wölbung des Gehgipses anpassen können. Beim Strammziehen des Gurtbandsystems werden deshalb die flexiblen Führungsstege an den Gehgips angepreßt und biegen sich über ihn. Dies gilt sowohl für Gehgipse, die ohne weiteres zwischen die Führungsstege passen, als auch für Gehgipse, die etwas breiter sind als der Abstand der Führungsstege. Im letzteren Fall können die Führungsstege dann nach außen ausweichen und werden beim Strammziehen des Gurtbandsystems wieder an dem Gehgips angepreßt. Insgesamt ergibt sich hierdurch eine deutliche Verbesserung in der Festlegung der Gehgipssohle am Gehgips, und zwar auch dann, wenn der Gehgips Überbreite hat.

Die Führungsstege sind an dem Sohlenkörper angeformt, bestehen also aus dessen Material. Damit sie sich der Krümmung des Gehgipses besser anpassen können, sollte die Flexibilität der Führungsstege nach oben hin zunehmen. Dies wird dadurch erreicht, daß die Dicke der Führungsstege und gegebenenfalls deren Erstreckung in Längsrichtung nach oben hin abnehmen. Die Führungsstege haben vorteilhafterweise in etwa eine Halbkreisform.

Durch eine solche Ausgestaltung des Sohlenkörpers kann jedoch noch eine weitere Verbesserung des Haltes erreicht werden.

Nach einem weiteren Merkmal der Erfindung ist vorgesehen, daß zwei Durchtritte, und zwar ein vorderer und ein hinterer, vorgesehen sind, wobei der hintere Durchtritt im Bereich vor der Fersenstütze angeordnet ist. Dies bedingt einen schräg nach oben gehenden Verlauf des Gurtbandsystems in diesem Bereich mit der Folge, daß der Gehgips dort zusätzlich seitengeführt wird und in diesem Bereich nicht von der Gehgipssohle abheben kann.

Es wird damit eine wesentlich bessere Festlegung erreicht als mit der Anordnung der hinteren Durchtritte in der Fersenstütze selbst. Gleichwohl kann diese Ausbildung der Fersenstütze verbesserten Halt geben.

In weiterer Ausgestaltung der Erfindung ist vorgesehen, daß das Gurtbandsystem nur ein einziges Gurtband aufweist, das die Durchtritte quer durchläuft, wobei jedoch die Gurtbandabschnitte außerhalb der Durchtritte und zwischen dem vorderen und hinteren Durchtritt diagonal und sich kreuzend von einer Seite zur anderen verlaufen und die beiden Enden außerhalb der Durchtritte über ein Verbindungsmittel verstellbar gekuppelt sind.

Diese diagonale Überkreuzführung des einzigen Gurtbandes oberhalb des Sohlenkörpers hat eine weitere wesentlich bessere Festlegung der Gehgipssohle zur Folge. Dies hängt offenbar damit zusammen, daß die über den Gehgips laufenden Abschnitte des Gurtbandes wesentlich länger sind als bei den bekannten Gurtbandsystemen mit zwei Gurtbändern und daß sie erheblich größere Anteile haben, mit denen sie an den Seiten des Gehgipses verlaufen. Hinzu kommt, daß die Gehgipssohle in der Handhabung wesentlich einfacher und sicherer ist. Durch Zug an dem aus dem einzigen Verbindungsmittel herausragenden Ende wird das Gurtband gleichmäßig über seine gesamte Länge an den Gehgips herangeführt und mit diesem verspannt. Es kann also nicht mehr - wie im Stand der Technik - passieren, daß das eine Gurtband zu lose und das andere zu fest angezogen wird. Für die Krankenhausträger ergibt sich der Vorteil, daß nur ein Gurtband vorgehalten und eingezogen werden muß.

Als Verbindungsmittel kommen insbesondere Klemmschnallen und Klettverschlüsse in Frage, aber auch einfache Schnallen mit Schnallstiften.

In der Zeichnung ist die Erfindung an Hand eines perspektivisch dargestellten Ausführungsbeispiels näher veranschaulicht. Sie zeigt die erfindungsgemäße Gehgipssohle (1), die im wesentlichen aus einem Sohlenkörper (2), einer hintenseitig vom Sohlenkörper (2) hochstehenden Fersenstütze (3) und einem Gurtband (4) besteht. Der Sohlenkörper (2) und die Fersenstütze (3) sind als einstückiges Kunststofformteil spritzgegossen. Die Oberseite (5) des Sohlenkörpers (2) weist eine Reihe von Vertiefungen - beispielhaft mit (6) bezeichnet - zur Materialersparnis auf. Die Unterseite (7) ist zur Verbesserung der Rutschfestigkeit geriffelt und teilweise gewölbt.

Der Sohlenkörper (2) weist einen vorderen Durchtritt (8) und einen hinteren Durchtritt (9) auf. Beide Durchtritte (8, 9) verlaufen quer durch den Sohlenkörper (2). Der vordere Durchtritt (8) besteht aus zwei Ösen (10̸, 11) und einer dazwischenliegenden Vertiefung (12) in der Oberseite (5). Der hintere Durchtritt (9) wird von vier Ösen (13, 14, 15, 16) gebildet, zwischen denen Vertiefungen (6) eingeformt sind.

Die Oberseite (5) des Sohlenkörpers (2) ist eben ausgebildet, hat jedoch neben den Vertiefungen (6) eine Längsnut (26) mit scharfen Seitenkanten, die ein seitliches Verrutschen des Gehgipses verhindern. Im Bereich des vorderen Durchtritts (8) stehen von den Seitenrändern (17, 18) in etwa halbkreisförmige Führungsstege (19, 20̸) hoch. Sie sind an dem Sohlenkörper (2) angeformt, wobei ihre Außenseiten mit den Seitenflächen des Sohlenkörpers (3) bündig sind. Die Führungsstege (19, 20̸) sind so dünn ausgebildet, daß sie flexibel, jedoch nicht biegeschlaff sind. Unter Flexibilität ist in diesem Zusammenhang eine Biegsamkeit zu verstehen, bei der die Führungsstege (19, 20̸) einerseits ein seitliches Herausrutschen des Gehgipses noch zuverlässig verhindern, andererseits sich jedoch an die außenseitige Wölbung des Gehgipses anpassen können. Ihre maximale Höhe gegenüber den Seitenwandungen (17, 18) beträgt im Ausführungsbeispiel 3,5 cm und ihre Dicke an der Basis 3 mm. Letztere nimmt nach oben hin auf 2 mm ab.

In die beiden Durchtritte (8, 9) ist das einzige Gurtband (4) eingezogen, und zwar in der Weise, daß die aus den beiden Ösen (10̸, 11) des vorderen Durchtritts (8) austretenden Gurtbandabschnitte (22, 23) diagonal und sich kreuzend nach hinten verlaufen und jeweils auf der anderen Seite des Sohlenkörpers (2) in den Durchtritt (9) eintreten. Der obenseitig verlaufende Gurtbandabschnitt (22) besteht aus den Enden des Gurtbandes (4). Das eine Ende weist eine Klemmschnalle (24) auf, die von dem anderen Ende des Gurtbandes (21) durchsetzt wird. Die Festlegung dieses Endes geschieht in der Klemmschnalle (24) durch eine Exenterverklemmung. Dies ermöglicht eine stufenlose Anpassung der Längen der Gurtbandabschnitte (22, 23) an den jeweils mit der Gehgipssohle (1) zu versehenden Gehgips. Durch Strammziehen des freien Endes des Gurtbandes (4) legen sich die Gurtbandabschnitte (22, 23) fest um die Führungsstege (19, 20̸) und den Gehgips. Da sie beim Austritt aus den Ösen (10̸, 11) des vorderen Durchtritts (8) über die Außenseite der flexiblen Führungsstege (19, 29) verlaufen, werden diese beim Strammziehen gegen den Gehgips herangebogen und passen sich an dessen Wölbung an. Dies geschieht auch dann, wenn der Gehgips eine etwas größere Breite hat als der Abstand der Führungsstege (19, 20̸).

Im vorderen Bereich ist in die Oberseite (5) des Sohlenkörpers (2) eine Quernut (25) eingeformt. Diese Quernut (25) erleichtert ein Abbrechen oder Absägen des davor liegenden Teils des Sohlenkörpers (2), wenn dies erwünscht ist.

## Patentansprüche

1. Gehgipssohle (1) mit einem Sohlenkörper (2), der mehrere Durchtritte (8, 9) zur Querführung eines darin eingezogenen Gurtbandsystems (4) zwischen den Seitenrändern (17, 18) des Sohlenkörpers (2) vorsieht, wobei die Seitenränder (17, 18) vorragend ausgebildet sind und wobei am hintenseitigen Ende eine nach oben vorstehende Fersenstütze mit vorragenden Seitenrändern angeformt ist, dadurch gekennzeichnet, daß von den Seitenrändern (17, 18) des Sohlenkörpers (2) im Bereich des vorderen Durchtritts (8) an den Sohlenkörper (2) angeformte Führungsstege (19, 20) hochstehen, deren Höhe gegenüber den Seitenrändern wenigstens 2,5 cm beträgt, deren Dicke nach oben hin abnimmt, die flexibel sind und über deren Außenseiten das Gurtbandsystem (4) nach oben verläuft.

2. Gehgipssohle nach Anspruch 1, dadurch gekennzeichnet, daß die Erstreckung der Führungsstege (19, 20) in Längsrichtung nach oben hin abnimmt.

3. Gehgipssohle nach Anspruch 1 oder 2, dadurch gekennzeichnet, daß die Führungsstege (19, 20) in etwa eine Halbkreisform haben.

4. Gehgipssohle nach einem der Ansprüche 1 bis 3, dadurch gekennzeichnet, daß zwei Durchtritte (8, 9), und zwar ein vorderer und ein hinterer, vorgesehen sind, wobei der hintere Durchtritt (9) im Bereich vor der Fersenstütze (3) angeordnet ist.

5. Gehgipssohle nach einem der Ansprüche 1 bis 4, dadurch gekennzeichnet, daß das Gurtbandsystem nur ein einziges Gurtband (4) aufweist, das die Durchtritte (8, 9) quer durchläuft, wobei jedoch die Gurtbandabschnitte (22, 23) außerhalb der Durchtritte (8, 9) und zwischen dem vorderen und hinteren Durchtritt (8, 9) diagonal und sich kreuzend von einer Seite zur anderen verlaufen und die beiden Enden außerhalb der Durchtritte (8, 9) über ein Verbindungsmittel (24) verstellbar gekuppelt sind.

6. Gehgipssohle nach Anspruch 5, dadurch gekennzeichnet, daß das Verbindungsmittel als Klemmschnalle (24) ausgebildet ist.

7. Gehgipssohle nach Anspruch 5, dadurch gekennzeichnet, daß das Verbindungsmittel als Klettverschluß ausgebildet ist.

## Claims

1. Walking cast sole (1) having a sole body (2) which provides a number of passages (8, 9) for guiding a strap system (4), introduced into the passages, across between the side edges (17, 18) of the sole body (2), the side edges (17, 18) being designed protruding, and an upwardly projecting heel support with protruding side edges being formed integrally on the rear end, characterized in that guide ridges (19, 20) formed integrally onto the sole body (2) rise vertically from the side edges (17, 18) of the sole body (2) in the area of the front passage (8), the height of the guide ridges (19, 20) relative to the side edges being at least 2.5 cm and their thickness decreasing towards the top, these guide ridges being flexible, and the strap system (4) extending upwards over their outer sides.

2. Walking cast sole according to Claim 1, characterized in that the extent of the guide ridges (19, 20) in the longitudinal direction decreases towards the top.

3. Walking cast sole according to Claim 1 or 2, characterized in that the guide ridges (19, 20) have approximately a semicircular shape.

4. Walking cast sole according to one of Claims 1 to 3, characterized in that two passages (8, 9) are provided, namely a front passage and a rear passage, the rear passage (9) being arranged in the area in front of the heel support (3).

5. Walking cast sole according to one of Claims 1 to 4, characterized in that the strap system has only a single strap (4) which runs transversely through the passages (8, 9), the strap sections (22, 23) outside the passages (8, 9) and between the front and rear passages (8, 9), however, extending from one side to the other diagonally and intersecting each other, and the two ends outside the passages (8, 9) being coupled in an adjustable manner via a connection means (24).

6. Walking cast sole according to Claim 5, characterized in that the connection means is designed as a strap buckle (24).

7. Walking cast sole according to Claim 5, characterized in that the connection means is designed as a Velcro fastener.

## Revendications

1. Semelle pour plâtre de marche (1) avec un corps de semelle (2) qui comprend plusieurs passages (8, 9) pour le guidage transversal d'un système à lanière (4) inséré dans ces passages entre les bords latéraux (17, 18) du corps de semelle (2), les bords latéraux (17, 18) étant formés en saillie et une pointe de talon se projetant vers le haut étant formée à l'extrémité postérieure avec des bords latéraux en saillie, caractérisée en ce que des languettes de guidage (19, 20) formées depuis les bords latéraux (17, 18) du corps de semelle (2) dans le domaine du passage avant (8) sur le corps de semelle (2) sont remontées vers le haut, leur hauteur par rapport aux bords latéraux étant d'au moins 2,5 cm, leur épaisseur diminuant vers le haut, en ce qu'elles sont flexibles et en ce que le système à lanière (4) s'étire vers le haut par les côtés externes de ces languettes.

2. Semelle pour plâtre de marche selon la revendication 1, caractérisée en ce que la dimension des languettes de guidage (19, 20) diminue dans le sens longitudinal vers le haut.

3. Semelle pour plâtre de marche selon la revendication 1 ou 2, caractérisée en ce que les languettes de guidage (19, 20) ont une forme approximativement semi-circulaire.

4. Semelle pour plâtre de marche selon l'une quelconque des revendications 1 à 3, caractérisée en ce que deux passages (8, 9) et en fait un passage avant et un passage arrière sont prévus, le passage arrière (9) étant disposé dans le domaine situé devant la pointe de talon (3).

5. Semelle pour plâtre de marche selon l'une quelconque des revendications 1 à 4, caractérisée en ce que le système à lanière ne présente qu'une seule lanière (4), qui passe à travers les passages (8, 9), les sections de la lanière (22, 23) passant cependant à l'extérieur des passages (8, 9) et entre le passage avant et le passage arrière (8, 9) en diagonale et en se croisant d'un côté vers l'autre, et les deux extrémités étant couplées à l'extérieur des passages (8, 9) par un moyen de connexion (24) de manière à pouvoir glisser.

6. Semelle pour plâtre de marche selon la revendication 5, caractérisée en ce que le moyen de connexion est formé en tant que boucle de serrage (24).

7. Semelle pour plâtre de marche selon la revendication 5, caractérisée en ce que le moyen de connexion est formé en tant que fermeture de type velcro.
